# EUROPEAN PATENT SPECIFICATION

(11) **EP 2 603 157 B1**
(45) Date of publication and mention of the grant of the patent: **24.05.2017**
(21) Application number: 11761409.9
(22) Date of filing: 02.08.2011
(51) Int. Cl.: A61B 90/98, A61F 13/44

(54) **METHOD FOR ATTACHING AN RF TAG TO A SPONGE ITEM**
VERFAHREN ZUR BEFESTIGUNG EINES RF-ETIKETTS AN EINEM SCHWAMMARTIKEL
PROCÉDÉ POUR FIXER UNE RADIO-ÉTIQUETTE À UN OBJET DE TYPE ÉPONGE

(30) Priority: 09.08.2010 US 852525
(43) Date of publication of application: 19.06.2013
(73) Proprietor: Haldor Advanced Technologies Ltd., 45319 Hod Hasharon (IL)
(72) Inventor: HALBERTHAL, Reuven, 44862 Tzur Igal (IL); POREMBA, Jacob, 74084 Nes Ziona (IL)
(74) Representative: Schmidt, Steffen
(86) International application number: PCT/IL2011/000625
(87) International publication number: WO 2012/020399

(56) References cited:
- EP-A2- 1 804 203
- DE-A1- 4 020 069
- GB-A- 2 472 025
- US-A- 6 026 818
- US-A1- 2002 185 212
- US-A1- 2005 049 564
- US-A1- 2006 232 407
- US-A1- 2008 024 278
- US-A1- 2009 212 913

## Description

### BACKGROUND OF THE INVENTION

### FIELD OF THE INVENTION

The present invention relates to attaching RF tags in general and to attaching RF tags to a sponge and gauze item in particular.

### DISCUSSION OF THE RELATED ART

There are many environments in which multiple tools and disposables are used, including for example operation rooms, hangars, garages, or the like.

An operation room is a facility in which intrusive operations are performed on patients. Typically, multiple people participate in an operation, using multiple tools, such as scalpels, forceps, and others, varying according to the surgery being performed.

Intensive efforts are invested in keeping track of all tools and disposables, in order to make sure no tool unintentionally remains inside the patient's body. Therefore, careful counting is performed before, during and after the operation. Counting the tools is a tedious job and requires intensive resources, including mental resources, personnel time and down-time of the operating room. Counting the tools towards the end of an operation also increases the time the patient's body is open with the associated risks. In addition, counting is not always error-free, and in too many cases tools end up being left within the patient's body, causing severe damages and even death.

One of the elements counted in an operation room is sponge items, such as gauze and laparotomy sponges. It is also desired to count the sponge items after an
operation to verify that none is left in the patient's tissues. Counting the sponge items can be performed by detecting X-ray detectable wires attached to the sponge items using an X-ray machine. Such machine generates radiation and cannot distinguish one sponge item from several items. As a result, after removing a sponge item from the patient's tissue, one still cannot verify that the patient's tissue is free from sponge items unless rescanning the patient again with X-ray machine.

To solve the above problem, RF tags may be attached to sponge items by sewing. The sewing may be performed manually, which is not cost-effective and time consuming. Manual sewing reduces the throughput of the attaching process and increases the costs of the machine that outputs the sponges from raw sheet of the sponge material. It is challenging to mechanize the sewing process, as there are many sizes and models of sponge items, on which the RF tags are to be attached. For example, a different machine is required for sewing an RF tag to a 30cm long sponge than for a 45cm long sponge.

There is thus a need in the art for a biocompatible and sterilization-resistant identification tag to be attached to a sponge, and an automated cost effective method for attaching the tag to the sponge.

US 2008/0024278 A1 discloses an interrogator, methods of discerning the presence of an object, and interrogation systems employing the same. The interrogation systems include multiple interrogators that communicate with a base command unit to track a location of an object. The object may be an RFID object (e.g., an object with an RFID tag), the interrogators employ signal processing techniques such as precharging the RFID object, and correlating a reference code with a reply code from the RFID object using selected techniques to increase a sensitivity of the interrogator, especially for adverse environments. The interrogation systems may include variations of metal instruments and sponges employed therewith.

### SUMMARY OF THE PRESENT INVENTION

According to the present invention, a method according to claim 1 of attaching a tag to a disposable item, wherein the disposable item is one or more of a sponge, a cotton wool item, a gauze pad or a laparotomy sponge, is provided. According to a further aspect of the present disclosure, a method of attaching a tag to a sponge item is disclosed, the method comprising obtaining a tag, obtaining a sponge item and attaching the tag to the disposable item using an adhesive material. The adhesive material is cured by ultraviolet radiation. The method further comprises applying the ultraviolet radiation on the tag attached on the disposable item.

In some cases, the tag is an RF tag. In some cases, the adhesive material is attached to the tag before the tag is attached to the disposable item. In some cases, the adhesive material is attached to the disposable item before the tag is attached to the disposable item.

In some cases, the method further comprises a step of folding the disposable item. In some cases, the adhesive material is attached to more than one layer of the folded disposable item. In some cases, the ultraviolet radiation cures the adhesive material in more than one layer of the folded disposable item.

In some cases, the step of attaching is performed before folding the disposable item. In some cases, the method further comprises a step of maneuvering the tag from tag storage to a working plate on which the disposable item is mounted. Optionally, the adhesive material is DYMAX Ultra Light-Weld 204-CTH-F.

According to still another aspect of the present disclosure, a system for attaching a tag to a sponge item is disclosed, the system comprising a maneuvering mechanism for maneuvering the tag towards the sponge item and an adhesive material storage unit for providing adhesive material attaching the tag and the sponge item. The system also comprises an ultraviolet radiation module for radiating ultraviolet radiation on the tag attached to the sponge item; wherein the ultraviolet radiation cures the adhesive material.

In some cases, the maneuvering mechanism maneuvers the tag to the adhesive material storage unit before mounting the tag on the sponge item.

### BRIEF DESCRIPTION

Exemplary non-limited embodiments of the disclosed subject matter will be described, with reference to the following description of the embodiments, in conjunction with the figures. The figures are generally not shown to scale and any sizes are only meant to be exemplary and not necessarily limiting. Corresponding or like elements are optionally designated by the same numerals or letters.
Fig. 1 shows a schematic illustration of an operation room, in which an identification and tracking system and method are required, according to exemplary embodiments of the subject matter;
Fig. 2 shows a top view of a production line environment for attaching a tag to a disposable item, according to exemplary embodiments of the subject matter;
Fig. 3 shows a method for attaching a tag to a disposable item, according to exemplary embodiments of the subject matter.

### DETAILED DESCRIPTION OF EXEMPLARY EMBODIMENTS

One technical challenge disclosed in the subject matter is to attach a tag to a sponge item while maintaining sterilization requirements, such as used in medical environments and hospitals.

One technical solution of the disclosed subject matter is a system and a method for attaching a tag to a sponge item. The tag can be an RF tag. The method comprises receiving a sponge item from a storage and mounting the sponge item on a working plate. The method further comprises receiving a tag to be soaked in a UV adhesive material and placed on the sponge item and placing the tag on the sponge item. The method also comprises a step of drying an adhesive material, attaching the tag to the disposable item. The drying is performed using Ultraviolet light emitted from a UV emitter.

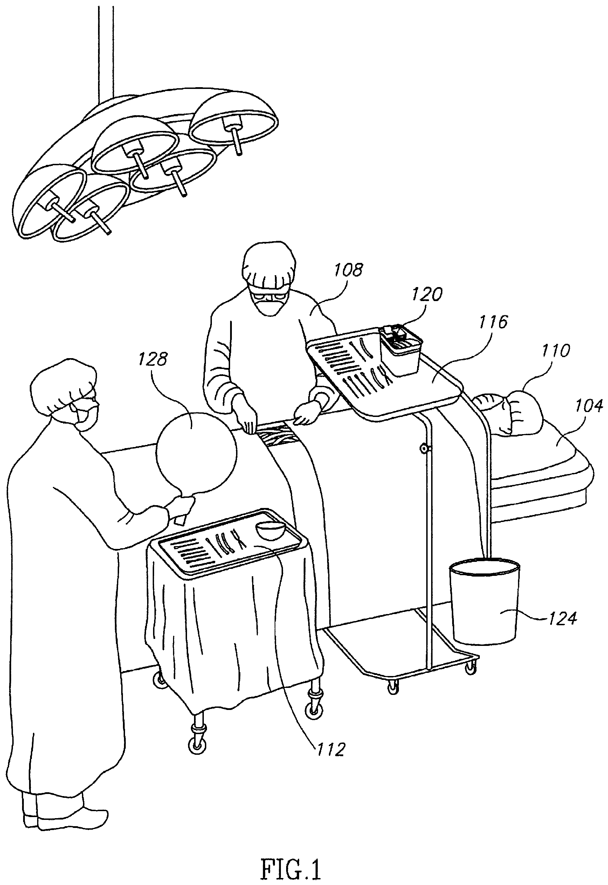

Fig. 1 shows a schematic illustration of an operation room, in which an identification and tracking system and method are required.

A typical operation room comprises an operation bed 104 on which a patient 110 being operated on lies. A surgeon 108 stands by patient 110 and operates on him. A circulating nurse who does not touch the sponges but only opens their wraps may place the sponges on instrument table 112. Surgeon 108 receives the sponges as required from a scrub nurse who takes the sponges from an instrument table 112.

Surgeon 108 or another team member can place the sponges on a moveable tray 116 placed above or near the patient 110, often called a Mayo. Each operation room typically has one or more instrument tables and one or more Mayos, depending on factors such as the complexity of the surgery, number of surgeons and other team members, personal preferences or others.

The team members retrieve clean sponges from one or more clean sponge bins or dispenser 120, and throw the used ones into one or more waste buckets 124.

Thus, at the end of the surgery, all sponges that were in the operation room prior to the surgery, should be on instrument table 112, on Mayo 116, in clean sponge bin 120 or in waste bucket 124 (collectively referred to as the "utilities").

The operation room is also equipped with a wand 128, which is also an antenna, and which is used for identifying and tracking items within the body of patient 110, by waving the wand near patient 110.

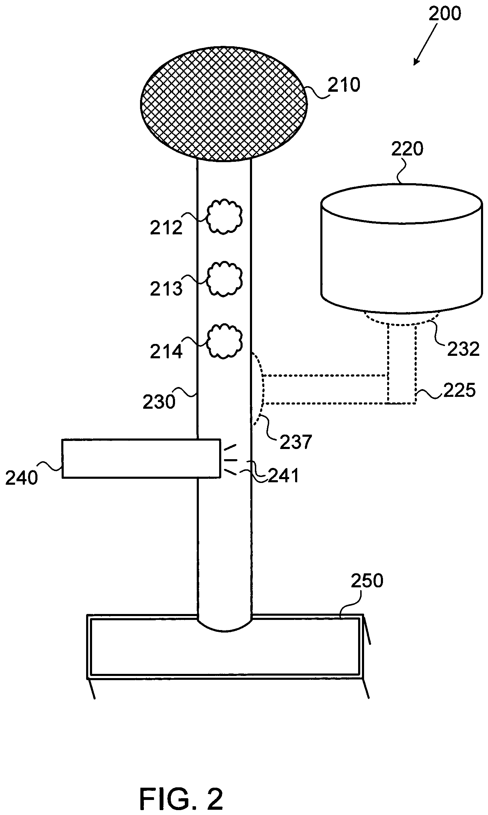

Fig. 2 shows a top view of a production line environment 200 for attaching a tag to a disposable item, according to exemplary embodiments of the subject matter. The environment 200 comprises a tag storage 220 for storing one or more tags to be attached to disposable items.

The environment 200 further comprises a disposable item storage 210 for storing disposable items. The disposable items may also be provided directly from a manufacturing machine or a production line of disposable items, which may be regarded as equivalent to the disposable item storage 210. The disposable item may be of one or more types, such as sponges, or cotton wool items. The disposable item may be a gauze pad, a laparotomy sponge and the like. The disposable item storage 210 may comprise two or more storage units for storing two or more types of disposable items.

The tag storage 220 may store the tags arranged in a tag stack. The tag storage 220 may comprise a mechanism for outputting one or more tags every predefined period, for example for 2 disposable items every 10 seconds.

The environment 200 may further comprise a working plate 230 on which the tag is attached to the disposable item. The working plate 230 may be a conveyor belt. The working plate 230 may be positioned between the disposable item storage 210 and an endpoint 250 to which the disposable items with an attached tag are provided. Optionally, disposable items are released from disposable item storage 210 and conveyed along working plate 230 to endpoint 250.

The environment 200 may further comprise a maneuvering mechanism 225 for maneuvering tags from the tag storage 220 to meet disposable items such as 212, 213, and 214 as they are being conveyed along working plate 230. Optionally, tag storage 220 has a tag release interface 232 that provides tags from tag storage 220 to maneuvering mechanism 225. Optionally, the maneuvering mechanism 225 moves tags from tag release interface 232, wherein the maneuver mechanism 225 obtains a tag, to a disposable item interface 237, wherein the maneuvering mechanism 225 delivers the tags to be mounted on the disposable item. The maneuvering mechanism 225 may contain one or more gripping arms (not shown) to receive the tag from the tag storage 220.

The tag is attached to the disposable item using an adhesive material that is UV curable. The adhesive material may be provided on the tag before or after the tag is obtained from the maneuvering mechanism 225. The adhesive material can be provided on the disposable item before attachment to the tag. The UV curable adhesive material may be DYMAX Ultra Light-Weld 204-CTH-F.

In an exemplary embodiment of the disclosed subject matter, the maneuvering mechanism 225 grips a tag, maneuvers the tag to a dispensing area of an adhesive material storage and places the tag containing the adhesive material on the disposable item. In some cases, the adhesive material is obtained only on one side of the tag, the side attached to the disposable item.

The disposable items such as 212, 213, and 214 may be positioned on the working plate 230 when the tags are mounted thereon. The disposable items may be folded in order to reduce the volume or surface area they consume. Attaching the tags may be done before folding or after.

The environment 200 further comprises an ultraviolet (UV) emitting device 240. The UV emitting device 240 emits UV radiation 241 on the tag attached to the disposable item as it passes by. The UV radiation 241 cures the adhesive material. The adhesive material is adapted to be cured by the UV radiation 241. The UV radiation 241 may be applied on a predefined area on the working plate 230 or applied generally to the direction of the tag attached to the disposable item.

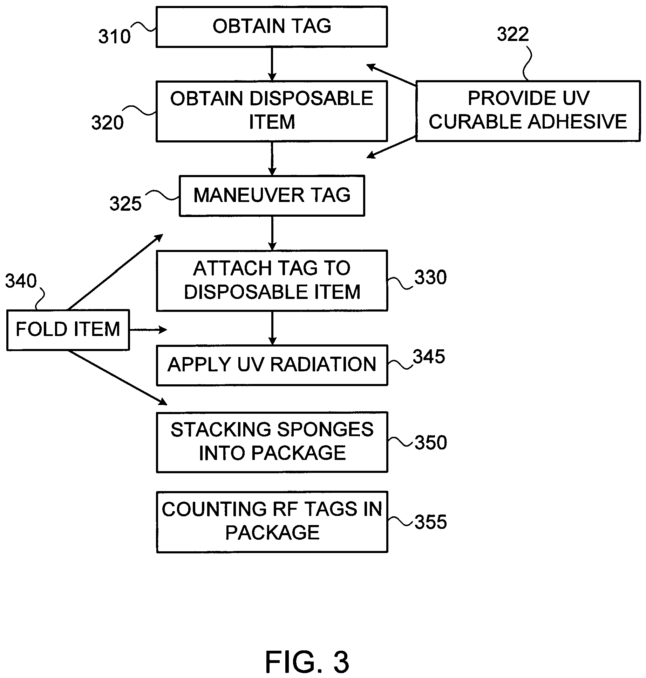

Figure 3 shows a method for attaching a tag to a disposable item, according to exemplary embodiments of the subject matter. In step 320, a disposable item is obtained. The disposable item may be obtained from a disposable item storage or from a user. As explained above the disposable item may be a sponge, cotton wool, and the like.

In step 310, a tag is obtained. The tag may contain a transmitting device, sending a signal to a computerized or electronic entity. Such computerized or electronic entity receives signals from one or more items in the environment and provides indications to the user. The tag may contain an RF transmitter or another transmitter that transmits wireless signals to the computerized or electronic entity.

In step 325, the tag is maneuvered from a tag storage to the disposable item. The maneuvering may be performed using a maneuvering mechanism 225. The tag can be held in one or more gripping arms while being maneuvered towards the disposable item. The maneuver can be made to a predefined area in the working plate 230 as shown in Fig. 2.

In step 330, the tag is attached to the disposable item. The attachment may be performed on a predefined area of the disposable item. In some cases, the maneuvering mechanism 225 comprises a control unit for determining the location in which the tag is released from the maneuvering mechanism 225.

In step 322, adhesive material is provided to attach the tag to the disposable item. The adhesive material is curable using ultraviolet radiation. The adhesive material may be, for example, DYMAX Ultra Light-Weld 204-CTH-F. The adhesive material may be provided on the tag. Alternatively, the adhesive material may be provided on the disposable item.

In step 340, the disposable item is folded. The folding may be performed before or after the tag is attached to the disposable item. The folding may be performed by a mechanical or electronic mechanism or by a person. The adhesive material may be in contact with more than one layer of the folded disposable item.

In step 345, ultraviolet radiation is applied on the disposable item attached to the tag. Such ultraviolet radiation may be in an amount of about 50 mW/cm² or
in a range of 50 mW/cm² - 10 W/cm². The ultraviolet radiation may be applied for a duration in a range of 0.5 to 2 seconds in order to cure the adhesive material on the disposable item. In other cases, the amount of ultraviolet radiation depends on the type of the adhesive material. The radiated ultraviolet radiation cures the adhesive material.

In step 350, one or more disposable items are packed into a package for delivery to a hospital or another entity using the disposable items. In step 355, the number of tags in each package are counted to verify the number of disposable items in each package.

## Claims

1. A method of attaching an RF tag to a disposable item (212), wherein the disposable item (212) is one or more of a sponge, a cotton wool item, a gauze pad or a laparotomy sponge, the method comprising:
obtaining an RF tag;
obtaining a disposable item (212);
placing the disposable item (212) on a working plate (230) in the form of a conveyor belt;
conveying the disposable item (212) to an attaching point;
attaching the RF tag to the disposable item (212) using a UV curable adhesive material;
applying a pre-determined amount of ultraviolet radiation (241) on the RF tag attached on the disposable item (212) to cure the UV curable adhesive material with the ultraviolet radiation (241) as the disposable item (212) passes by an ultraviolet emitting device (240), wherein the pre-determined amount is selected depending on the type of UV curable adhesive material;
packing one or more of the disposable (212) items into a package for delivery, and
using a computerised or electronic entity with an antenna to communicate with the RF tags for counting the number of RF tags in the package to verify that the number of disposable items (212) in the package is as expected.

2. The method according to claim 1, wherein the UV curable adhesive material is attached to the RF tag before the RF tag is attached to the disposable item (212).

3. The method according to claim 1, wherein the UV curable adhesive material is attached to the disposable item (212) before the RF tag is attached to the disposable item (212).

4. The method according to claim 1, further comprises a step of folding the disposable item (212).

5. The method according to claim 4, wherein the UV curable adhesive material is attached to more than one layer of the folded disposable item (212).

6. The method according to claim 4, wherein the ultraviolet radiation cures the UV curable adhesive material in more than one layer of the folded disposable item (212).

7. The method according to claim 4, wherein the step of attaching is performed before folding the disposable item (212).

8. The method according to claim 1, further comprising a step of maneuvering the RF tag from a tag storage (220) to the working plate (230) on which the disposable item (212) is mounted.

## Patentansprüche

1. Verfahren zum Befestigen eines RF-Etiketts an einem Einwegartikel (212), wobei der Einwegartikel (212) eines oder mehrere von einem Schwamm, einem Watteartikel, einem Mulltupfer oder einem Bauchtuch ist, das Verfahren umfassend:
Erhalten eines RF-Etiketts;
Erhalten eines Einwegartikels (212);
Platzieren des Einwegartikels (212) auf eine Arbeitsplatte (230) in Form eines Förderbandes;
Fördern des Einwegartikels (212) zu einem Befestigungspunkt;
Befestigen des RF-Etiketts an dem Einwegartikel (212) unter Verwendung eines UV-härtbaren Klebstoffes;
Anwenden einer festgelegten Menge an ultravioletter Strahlung (241) auf das an dem Einwegartikel (212) befestigte RF-Etikett, um den UV-härtbaren Klebstoffe mit der ultravioletten Strahlung (241) zu härten, während der Einwegartikel (212) ein ultraviolett emittierendes Gerät (240) passiert, wobei die festgelegte Menge abhängig von dem Typ des UV-härtbaren Klebstoffes gewählt ist;
Packen eines oder mehrerer der Einwegartikel (212) in ein Paket für die Lieferung; und
Verwenden einer computergestützten oder elektronischen Einheit mit einer Antenne, um mit den RF-Etiketten zum Zählen der Anzahl von RF-Etiketten in der Packung zu kommunizieren, um zu verifizieren, dass die Anzahl von Einwegartikeln (212) in der Packung wie erwartet ist.

2. Verfahren nach Anspruch 1, wobei der UV-härtbare Klebstoff an dem RF-Etikett befestigt wird, bevor das RF-Etikett an dem Einwegartikel (212) befestigt wird.

3. Verfahren nach Anspruch 1, wobei der UV-härtbare Klebstoff an dem Einwegartikel (212) befestigt wird, bevor das RF-Etikett an dem Einwegartikel (212) befestigt wird.

4. Verfahren nach Anspruch 1, ferner umfassend einen Schritt des Faltens des Einwegartikels (212).

5. Verfahren nach Anspruch 4, wobei der UV-härtbare Klebstoff an mehr als einer Lage des gefalteten Einwegartikels (212) befestigt wird.

6. Verfahren nach Anspruch 4, wobei die ultraviolette Strahlung den UV-härtbaren Klebstoff in mehr als einer Lage des gefalteten Einwegartikels (212) härtet.

7. Verfahren nach Anspruch 4, wobei der Schritt des Befestigens vor dem Falten des Einwegartikels (212) ausgeführt wird.

8. Verfahren nach Anspruch 1, ferner umfassend einen Schritt des Manövrierens des RF-Etiketts von einem Etikettendepot (220) zu der Arbeitsplatte (230) auf der der Einwegartikel (212) angebracht wird.

## Revendications

1. Procédé de fixation d'une étiquette RF sur un article jetable (212), dans lequel l'article jetable (212) est un ou plusieurs parmi une éponge, un article en coton hydrophile, un tampon de gaze ou une éponge de laparotomie, le procédé comprenant :
l'obtention d'une étiquette RF ;
l'obtention d'un article jetable (212) ;
la mise en place de l'article jetable (212) sur une plaque de travail (230) sous la forme d'une courroie transporteuse ;
le transport de l'article jetable (212) jusqu'à un point de fixation ;
la fixation de l'étiquette RF sur l'article jetable (212) en utilisant un matériau adhésif durcissable aux UV ;
l'application d'une quantité prédéterminée de rayonnement ultraviolet (241) sur l'étiquette RF fixée sur l'article jetable (212) pour durcir le matériau adhésif durcissable aux UV avec le rayonnement ultraviolet (241) alors que l'article jetable (212) passe par un dispositif (240) d'émission d'ultraviolets, dans lequel la quantité prédéterminée est choisie en fonction du type de matériau adhésif durcissable aux UV;
le conditionnement d'un ou plusieurs des articles jetables (212) dans un emballage pour livraison ; et
l'utilisation d'une entité informatisée ou électronique avec une antenne pour communiquer avec les étiquettes RF pour compter le nombre d'étiquettes RF dans l'emballage pour vérifier que le nombre d'articles jetables (212) dans l'emballage est tel qu'attendu.

2. Procédé selon la revendication 1, dans lequel le matériau adhésif durcissable aux UV est fixé à l'étiquette RF avant que l'étiquette RF ne soit fixée à l'article jetable (212).

3. Procédé selon la revendication 1, dans lequel le matériau adhésif durcissable aux UV est fixé à l'article jetable (212) avant que l'étiquette RF ne soit fixée à l'article jetable (212).

4. Procédé selon la revendication 1, comprenant en outre une étape de pliage de l'article jetable (212).

5. Procédé selon la revendication 4, dans lequel le matériau adhésif durcissable aux UV est fixé à plus d'une couche de l'article jetable (212) plié.

6. Procédé selon la revendication 4, dans lequel le rayonnement ultraviolet durcit le matériau adhésif durcissable aux UV dans plus d'une couche de l'article jetable (212) plié.

7. Procédé selon la revendication 4, dans lequel l'étape de fixation est mise en oeuvre avant le pliage de l'article jetable (212).

8. Procédé selon la revendication 1, comprenant en outre une étape de manoeuvre de l'étiquette RF d'un stockage (220) d'étiquettes jusqu'à la plaque de travail (230) sur laquelle l'article jetable (212) est monté.
